# EUROPÄISCHE PATENTANMELDUNG

(11) **EP 3 257 496 A1**
(43) Veröffentlichungstag der Anmeldung: **20.12.2017**
(21) Anmeldenummer: 16174313.3
(22) Anmeldetag: 14.06.2016
(51) Int. Cl.: A61K 8/23, A61K 8/36, A61K 8/46, A61K 8/73, A61K 8/81, A61Q 5/02, A61Q 5/12

(54) **WÄSSRIGE TENSID-ZUSAMMENSETZUNGEN**

(71) Anmelder: BASF SE, 67056 Ludwigshafen am Rhein (DE)
(72) Erfinder: Brunn, Claudia, 40597 Duesseldorf (DE); Behler, Ansgar, 46240 Bottrop (DE)
(74) Vertreter: BASF IP Association

(57) **Zusammenfassung**

Vorgeschlagen werden wässrige Tensid-Zusammensetzungen enthaltend jeweils ein oder mehrere alpha-Sulfofettsäuredisalze, Sulfoketone, spezielle kationische Polymere, Seifen, anorganische Salze der Schwefelsäure und Wasser, wobei die Strukturen der genannten Verbindungen sowie einzuhaltende Randbedingungen den Patentansprüchen entnommen werden können. Diese Zusammensetzungen weisen ein gutes Schaumvermögen und eine angenehme Sensorik des Schaumes sowie eine gute Hautverträglichkeit auf und eignen sich für kosmetische Mittel sowie Wasch- und Reinigungsmittel.

## Beschreibung

### Gebiet der Erfindung

Die vorliegende Erfindung betrifft wässrige Tensid-Zusammensetzungen mit einem Gehalt an alpha-Sulfofettsäuredisalzen, Sulfoketonen, Seifen, anorganische Salze der Schwefelsäure und kationische Polymeren.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel ein gutes Schaumvermögen und eine angenehme Sensorik des Schaumes erwünscht. Des Weiteren besteht ein Bedarf an anionischen Tensiden, die zumindest teilweise aus biogenen Quellen und speziell auch nachwachsenden Rohstoffen hergestellt werden können.

WO-A-2008/155072 offenbart kosmetische Zubereitungen enthaltend a) mindestens ein Tensid ausgewählt aus anionischen, zwitterionischen oder amphoteren Tensiden, b) eine Mikroemulsion und c) mindestens ein kationisches Polymer.

EP-B2-971,689 offenbart wässrige pflegende Haarbehandlungsmittel enthaltend als Pflegewirkstoffe eine Mischung aus a) Sulfatierungs- und/oder Sulfonierungsprodukten von ungesättigten und/oder gesättigten Carbonsäuren und/oder Hydroxycarbonsäuren und b) Fettalkoholen, die dadurch gekennzeichnet sind, dass der pH-Wert für Shampoos, Kuren und Spülungen unter 4 liegt und bei direktziehenden Farbstoffen oder Vorprodukten für Oxidationsfarbstoffe enthaltenden Mitteln im Bereich von 6 bis 11 liegt (Anspruch 1). Optional können die Mittel ein kationisches Polymer enthalten (vergl. Anspruch 9, Absatz [0039]). In Absatz [0058] wird die Rezeptur für ein wässriges Haarfärbemittel (E5) offenbart, die außer Wasser zwölf Komponenten enthält, wobei zwei dieser Komponenten Laurin-/Myristinsäure-di-Na-Salz und Polymer JP®400 sind, wobei es sich gemäß den Angaben in der Legende der Tabelle bei letzterem um eine quaternierte Hydroxyethylcellulose handelt (INCI-Bezeichnung: Polyquaternium-10). Sulfoketone werden in der EP-B2-971,689 nicht thematisiert.

EP-A2-281,027 offenbart die Verwendung von Alpha-Sulfofettsäure-Salzen als Zusatz zur Verbesserung der Hautverträglichkeit und des Hautgefühls von wässrigen Tensidzusammensetzungen auf Basis von oberflächenaktiven Alkylethersulfaten, Alpha-Olefinsulfonaten, Sulfobernsteinsäuremonoestem und sekundären Alkansulfonaten. Neben den genannten obligatorischen Tensidkomponenten können auch weitere oberflächenaktive Substanzen enthalten sein. Die Verbesserung der Pflegeeigenschaften wird an Hand verbesserter Pflegewirkung am Haar (Reduzierung der Nasskämmarbeit) nachgewiesen. Kationische Polymere werden in der EP-A-281,027 nicht thematisiert.

### Beschreibung der Erfindung

Die Aufgabe der vorliegenden Erfindung hat darin bestanden, wässrige Tensid-Zusammensetzungen bereitzustellen, die eine hervorragende Pflegeleistung an Haut und Haar erzielen.

Gegenstand der Erfindung sind zunächst wässrige Tensid-Zusammensetzungen enthaltend
- ein oder mehrere **alpha-Sulfofettsäuredisalze (A)** der allgemeinen Formel (I),

   R¹CH(SO₃M¹)COOM² (I),

   worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
- ein oder mehrere **Sulfoketone (B),** die ausgewählt sind aus den Verbindungen (F) und den Verbindungen (G), wobei die Verbindungen (F) die allgemeine Formel (VI)

   R⁶CH₂-CO-CHR⁷(SO₃M⁸) ( VI),

   aufweisen, worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine,
   und wobei die Verbindungen (G) die allgemeine Formel (VII)

   (SO₃M⁹)R⁸CH-CO-CHR⁹(SO₃M¹⁰) (VII),

   aufweisen, worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine,

- ein oder mehrere **kationische Polymere (X)** ausgewählt aus der Gruppe der Polymere mit den INCI-Bezeichnungen Polyquaternium-6, Polyquaternium-7, Polyquaternium-10, Polyquaternium-11, Polyquaternium-16, Polyquaternium-22, Polyquaternium-24, Polyquaternium-32, Polyquaternium-37, Polyquaternium-39, Polyquaternium-44, Polyquaternium-46, Polyquaternium-47, Polyquaternium-53, Polyquaternium-67, Polyquaternium-68, Polyquatemium-72, Polyquaternium-74, Polyaclylamidopropyltrimonium Chloride, Polymethacrylamidopropyltrimonium Chloride, Acrylamidopropyltrimonium Chloride/Acrylamide Copolymer, Guar Hydroxypropyltrimonium Chloride, Cassia Hydroxypropyltrimonium Chloride und Starch Hydroxypropyltrimonium Chloride,
- ein oder mehrere **Verbindungen (C)** der allgemeinen Formel (III)

   R⁴COOM⁵ (III)

   wobei der Rest R⁴ einen linearen oder verzweigten Alkyl- oder Alkenylrest mit 7 bis 19 C-Atomen bedeutet und der Reste M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin, • ein oder mehrere **anorganische Salze der Schwefelsäure (D)** der allgemeinen Formel (IV)

   (M⁶)₂SO₄ (IV)

   wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin,
- **Wasser,**
wobei folgende Maßgabe gilt:
- Sofern die wässrigen Tensid-Zusammensetzungen ein oder mehrere Estersulfonate (E) der allgemeinen Formel (V),

   R²CH(SO₃M⁷)COOR³ (V)

   worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr - und insbesondere zu 90 Gew.-% oder mehr - vorliegen müssen.

### Zu den Verbindungen (A)

Die Verbindungen (A), die im Rahmen der vorliegenden Erfindung als **alpha-Sulfofettsäuredisalze** bezeichnet werden, sind für die erfindungsgemäßen wässrigen Tensid-Zusammensetzungen obligatorisch. Sie haben die oben angegebene Formel (I)

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine. Dabei gelten für die Verbindungen (A) die oben genannten Bedingungen:
In einer Ausführungsform beträgt der Anteil der Verbindungen (A), bei denen der Rest R¹ ein Alkenylrest ist, - bezogen auf die Gesamtmenge der Verbindungen (A) in den wäßrigen Tensid-Zusammensetzungen - bei 3 Gew.-% oder weniger.

Bezüglich der Reste M¹ und M² besonders bevorzugte Alkanolamine werden dabei ausgewählt aus der Gruppe Monoethanolamin, Diethanolamin, Triethanolamin und Mono-Isopropanolamin.

In einer bevorzugten Ausführungsform bedeutet der Rest R¹ in der Formel (I) einen gesättigten, linearen Alkylrest mit 10 bis 16 C-Atomen, wobei in Bezug auf die Verbindungen (A) gilt, dass der Anteil der Verbindungen (A), bei denen der Rest R¹ ein Decyl- und/oder ein Dodecylrest ist, - bezogen auf die Gesamtmenge der Verbindungen (A) - bei 70 Gew.-% oder mehr und insbesondere bei 90 Gew.-% oder mehr liegt.

Vorzugsweise werden die Reste M¹ und M² in der Formel (I) ausgewählt aus der Gruppe H (Wasserstoff) und Na (Natrium).

Die Verbindungen (A) können nach allen dem Fachmann einschlägig bekannten Methoden hergestellt werden. Eine besonders bevorzugte Methode der Herstellung ist dabei die Sulfierung der entsprechenden Carbonsäuren. Dabei setzt man die entsprechenden Carbonsäure und insbesondere die entsprechenden Fettsäuren mit gasförmigem Schwefeltrioxid um, wobei man das Schwefeltrioxid vorzugsweise in einer Menge einsetzt, dass das molare Verhältnis von SO₃ zu Fettsäure im Bereich von 1,0 : 1 bis 1,1 : 1 liegt. Die so erhaltenen Rohprodukte, die saure Sulfierprodukte darstellen, werden anschließend partiell oder vollständig neutralisiert, wobei eine vollständige Neutralisation mit wässriger NaOH bevorzugt ist. Gewünschtenfalls können auch Reinigungsschritte und/oder eine Bleiche (zur Einstellung der gewünschten hellen Farbe der Produkte) vorgenommen werden.

In einer besonders bevorzugten Ausführungsform werden die Verbindungen (A) in technischer Form eingesetzt. Dies bedeutet, dass man die entsprechenden Carbonsäuren, insbesondere native Fettsäure, mit gasförmigem Schwefeltrioxid sulfiert, wodurch nach partieller oder vollständiger Neutralisation der entstehenden sauren Sulfierprodukte ein Gemisch der Verbindungen (A), (C) und (D) resultiert. Durch entsprechende Einstellungen der Reaktionsparameter (insbesondere Mol-Verhältnis von Carbonsäure und Schwefeltrioxid sowie Reaktionstemperatur) lässt sich das Verhältnis der Verbindungen (A), (C) und (D) steuern. Die Verbindungen (C) und (D) sind unten beschrieben.

Im Rahmen der vorliegenden Erfindung sind solche technischen Mischungen der alpha-Sulfofettsäuredisalze bevorzugt, die wie folgt zusammengesetzt sind:
- Der Gehalt an (A) liegt im Bereich von 60 bis 100 Gew.-%,
- der Gehalt an (C) liegt im Bereich von 0 bis 20 Gew.-%,
- der Gehalt an (D) liegt im Bereich von 0 bis 20 Gew.-%,
mit der Maßgabe, dass die Summe der Komponenten (A), (C) und (D) in dieser Mischung 100 Gew.-% beträgt.

### Zu den Verbindungen (B)

Wie oben ausgeführt enthalten die erfindungsgemäßen wässrigen Tensid-Zusammensetzungen neben den Verbindungen (A) und Wasser ein oder mehrere **Sulfoketone (B),** die ausgewählt werden aus den Verbindungen (F) und (G).

Die **Verbindungen (F)** haben die allgemeine Formel (VI)

R⁶CH₂-CO-CHR⁷(SO₃M⁸) (VI),

worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (F) werden im Rahmen der vorliegenden Erfindung als Mono-Sulfo-Ketone bezeichnet.

In einer bevorzugten Ausführungsform bedeuten die Reste R⁶ und R⁷ in der Formel (VI) - unabhängig voneinander - einen gesättigten, linearen Rest mit 10 bis 16 C-Atomen, wobei in Bezug auf die Verbindungen (F) gilt, dass der Anteil der Verbindungen (F), bei denen die Reste R⁶ und R⁷ einen Decyl- und/oder ein Dodecylrest bedeuten, - bezogen auf die Gesamtmenge der Verbindungen (F) - bei 70 Gew.-% oder mehr und vorzugsweise bei 90 Gew.-% oder mehr liegt. In einer Ausführungsform wird der Rest M⁸ in der Formel (VI) ausgewählt aus der Gruppe H und Na.

Die **Verbindungen (G)** haben die allgemeine Formel (VII)

(SO₃M⁹)R⁸CH-CO-CHR⁹(SO₃M¹⁰) (VII),

worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (G) werden im Rahmen der vorliegenden Erfindung als Di-Sulfo-Ketone bezeichnet.

In einer bevorzugten Ausführungsform bedeuten die Reste R⁸ und R⁹ in der Formel (VII) - unabhängig voneinander - einen gesättigten, linearen Rest mit 10 bis 16 C-Atomen, wobei in Bezug auf die Verbindungen (G) gilt, dass der Anteil der Verbindungen (G), bei denen die Reste R⁸ und R⁹ einen Decyl- und/oder ein Dodecylrest bedeuten, - bezogen auf die Gesamtmenge der Verbindungen (G) - bei 70 Gew.-% oder mehr und vorzugsweise bei 90 Gew.-% oder mehr liegt. In einer Ausführungsform werden die Reste M⁹ und M¹⁰ in der Formel (VII) ausgewählt aus der Gruppe H und Na.

Die Herstellung der Verbindungen (F) und (G) unterliegt keinen besonderen Einschränkungen und sie können nach allen dem Fachmann bekannten Methoden hergestellt werden.

In einer Ausführungsform werden die Verbindung (F) und (G) durch Sulfonierung entsprechender Ketone mit gasförmigem Schwefeltrioxid hergestellt, wie in der deutschen Offenlegungsschrift DE-A-42,20,580 beschrieben.

In einer anderen Ausführungsform geht man zur Herstellung der Verbindungen (F) und (G) von Fettsäuren aus. Dabei führt man die Sulfierung von flüssigen Fettsäuren mit gasförmigem Schwefeltrioxid so durch, dass dabei neben Disalzen (A) auch die Verbindungen (F) und (G) entstehen, was dadurch realisiert werden kann, dass man die Sulfierung wie folgt durchführt: Das Verhältnis der Rohstoffe Fettsäure, die auch in Form von Gemischen von Fettsäuren unterschiedlicher Kettenlänge eingesetzt werden können, und Schwefeltrioxid wird so eingestellt, dass man 1,0 bis 1,5 mol und insbesondere 1,0 bis 1,25 mol SO₃ pro mol Fettsäure(n) einsetzt. Die Fettsäuren werden dabei mit einer Vorlagetemperatur im Bereich von 70 bis 100 °C in den Reaktor eingebracht. Nach der Sulfierung wird das erhaltene flüssige Sulfierprodukt in einer temperierten Nachreaktionsschlange für 5 bis 20 Minuten auf dieser Temperatur gehalten und gealtert. Anschließend erfolgt die Neutralisation mit einer wässrigen Base, vorzugsweise Natriumhydroxid, in der Regel bei einem pH-Wert im Bereich von 5 bis 10, insbesondere von 5 bis 7. Im Anschluss kann eine saure Bleiche - der pH-Wert wird hierbei auf einen Wert von 7 oder weniger eingestellt - mit Wasserstoffperoxid durchgeführt werden.

### Zu den Verbindungen (X)

Die Verbindungen (X), die im Rahmen der vorliegenden Erfindung als **kationische Polymere** bezeichnet werden, sind für die erfindungsgemäßen wässrigen Tensid-Zusammensetzungen obligatorisch. Die Verbindungen (X) werden dabei ausgewählt aus der Gruppe der kationischen Polymere mit den INCI-Bezeichnungen Polyquaternium-6, Polyquaternium-7, Polyquaternium-10, Polyquaternium-11, Polyquaternium-16, Polyquaternium-22, Polyquaternium-24, Polyquaternium-32, Polyquaternium-37, Polyquaternium-39, Polyquaternium-44, Polyquaternium-46, Polyquatemium-47, Polyquaternium-53, Polyquaternium-67, Polyquaternium-68, Polyquaternium-72, Polyquaternium-74, Polyaclylamidopropyltrimonium Chloride, Polymethacrylamidopropyltrimonium Chloride, Acrylamidopropyltrimonium Chloride/Acrylamide Copolymer, Guar Hydroxypropyltrimonium Chloride, Cassia Hydroxypropyltrimonium Chloride und Starch Hydroxypropyltrimonium Chloride.

In einer besonders bevorzugten Ausführungsform werden die Verbindungen (X) ausgewählt aus der Gruppe der kationischen Polymere mit der INCI-Bezeichnung Polyquaternium-6, Polyquaternium-7, Polyquaternium-10 und Guar Hydroxypropyltrimonium Chloride.

### Zu den Verbindungen (C)

Die Verbindungen (C) sind für die erfindungsgemäßen wässrigen Tensidzusammensetzungen obligatorisch. Die Verbindungen (C) haben die allgemeine Formel (III)

R⁴COOM⁵ (III)

In der Formel (III) bedeutet der Rest R⁴ einen linearen oder verzweigten Alkyl- oder Alkenylrest mit 7 bis 19 C-Atomen und der Reste M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

### Zu den Verbindungen (D)

Die Verbindungen (D), die im Rahmen der vorliegenden Erfindung als anorganische Salze der Schwefelsäure (D) bezeichnet werden, sind für die erfindungsgemäßen wässrigen Tensidzusammensetzungen obligatorisch. Die Verbindungen (D) haben die allgemeine Formel (IV)

(M⁶)₂SO₄ (IV)

wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin.

Gewünschtenfalls können die erfindungsgemäßen wässrigen Tensid-Zusammensetzungen zusätzlich ein oder mehrere weitere Tenside enthalten, die strukturell nicht zu den oben genannten Verbindungen (A), (B), (X), (C) oder (D) zählen. Bei diesen Tensiden kann es sich um anionische, kationische, nichtionische oder amphotere Tenside handeln.

### Bevorzugte Ausführungsformen

In einer Ausführungsform liegt der Gehalt der wässrigen Tensid-Zusammensetzungen an den Verbindungen (A) - bezogen auf die gesamte wässrige Tensid-Zusammensetzung - bei mindestens 2 Gew.-%, vorzugsweise im Bereich von 2 bis 50 Gew.-%, insbesondere im Bereich von 5 bis 20 Gew.-% und besonders bevorzugt im Bereich von 6 bis 12 Gew.-%.

In einer Ausführungsform liegt der Gehalt der wässrigen Tensid-Zusammensetzungen an den Verbindungen (X) - bezogen auf die gesamte wässrige Tensid-Zusammensetzung - bei mindestens 0,01 Gew.-%, vorzugsweise im Bereich von 0,01 bis 5 Gew.-% und insbesondere bei mindestens 0,05 bis 3 Gew.-%.

In einer bevorzugten Ausführungsform weisen die wässrigen Tensid-Zusammensetzungen einen pH-Wert im Bereich von 4 bis 6 auf.

### Verwendung der Zusammensetzungen

Ein weiterer Erfindungsgegenstand ist die Verwendung der oben genannten erfindungsgemäßen Zusammensetzungen für kosmetische Mittel. Dabei sind insbesondere solche kosmetische Mittel besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen.

Die oben genannten erfindungsgemäßen Zusammensetzungen können aber auch in Wasch- und Reinigungsmitteln eingesetzt werden. Dabei sind im Hinblick auf Reinigungsmittel insbesondere Mittel mit niedrigem pH-Wert zur Reinigung harter Oberflächen bevorzugt, wie Bad- und WC-Reiniger und dergleichen, sowie für Reinigungs- und/oder Duftgele zur Anwendung in sanitären Einrichtungen.

### Beispiele

### Eingesetzte Substanzen

**VE-Wasser** = vollentsalztes Wasser
**DSSK:** Zusammensetzung auf Basis von alpha-Sulfofettsäuredisalz technischer Qualität auf Basis von nativer C_{12/14}-Fettsäure (Gewichtsverhältnis C₁₂:C₁₄ = 70:30) und Sulfoketonen; Die Zusammensetzung von "DSSK" ist wie folgt: 67,6 Gew.-% Alpha-Sulfo-C_{12/14}-Fettsäure-DiNatriumsalz (Verbindung A), 14,5 Gew.-% C_{12/14}-Fettsäure-Natriumsalz (Verbindung C), 14,7 Gew.-% Natriumsulfat (Verbindung D), 1,0 Gew.-% 11-Sulfo-Tricosan-12-on Natriumsalz (Verbindung F), 0,6% Gew.-% 11,13-Di-Sulfo-Tricosan-12-on Di-Natriumsalz (Verbindung G) und 1,6 Gew.-% Wasser. Der Aktivgehalt, der zur Einwaage verwendet wird, berechnet sich als Summe der Gew.-% von Verbindung (A), (F) und (G).
**Texapon N70:** Sodium Laureth Sulfate (INCI-Bezeichnung), 70 Gew.-% Aktivsubstanz (Fa. BASF PCN)
**Dehyton PK45:** Cocamidopropyl Betaine (INCI- Bezeichnung), 70 Gew.-37% Aktivsubstanz (Fa. BASF PCN)
**Plantacare 1200 UP:** Lauryl Glucoside (INCI- Bezeichnung), 70 Gew.-52% Aktivsubstanz (Fa. BASF PCN)
**Ucare Polymer JR-400:** Polyquaternium-10 (INCI- Bezeichnung), (Fa. Dow)

### Mess- und Prüfmethoden

**pH-Wert:** Unter Einsatz eines handelsüblichen pH-Meters wurde der pH-Wert direkt in der wässrigen Formulierung gemessen.

**Rest-Nasskämmarbeit:** Die Vorbereitung der Haartressen (Europäisches Haar, 12cm/g, International Hair Importers & Products, USA) erfolgte in einem automatisierten System. Dabei wurden die folgenden Schritte durchlaufen: Zunächst wurden die Tressen zur Reinigung 15 Minuten lang in eine Sodium Laureth Sulfat-Lösung (6% Aktivsubstanz, pH 6.5) gelegt, danach mit Wasser abgespült und anschließend durch Eintauchen in Wasser (3 x 2 Minuten lang) nachgespült. Danach wurden die Haartressen zur definiert geschädigt indem sie mittels Wasserstoffperoxid gebleicht (5% H₂O₂, pH 9.4, 20 Minuten) und anschließend nochmals gründlich mit Wasser ausgespült wurden. Zum Schluss erfolgte die Trocknung in einem Warmluftstrom (30 Minuten, ca. 55°C).

Die Messung der Kämmarbeit erfolgte in einem Robotersystem, wobei zur Ermittlung eines Referenzwertes die Tressen zunächst vor Behandlung mit der wässrigen Tensid-Zusammensetzung (= Formulierung) vermessen wurden. Die Haartressen wurden dazu zunächst 30 Minuten in Wasser (38°C) gequollen, 2 Mal vorgekämmt und dann 21 Mal vom Kämmroboter gekämmt und dabei die im Mittel auftretende Kämmkraft bestimmt. Danach wurden die Haartressen 2 Mal für jeweils 5 Minuten mit der Formulierung benetzt (0,25g pro 1g Haar) und anschließend jeweils 1 Minute lang mit Wasser ausgespült. Anschließend wurden sie wiederum, 2 Mal vorgekämmt und dann 21 Mal gekämmt und die dabei im Mittel auftretende Kämmkraft bestimmt. Zum Vergleich der Pflegewirkung der Formulierungen wird jeweils die Rest-Nasskämmarbeit (in %) verglichen. Sie wird berechnet nach der Formel: (Kämmarbeit nach Behandlung) / (Kämmarbeit vor Behandlung) x 100%. Je stärker die Kämmarbeit durch die Behandlung reduziert wird, sprich je kleiner der Wert der Rest-Nasskämmarbeit ist, desto besser ist die Pflegeleistung der eingesetzten Formulierung am Haar zu bewerten.

### Beispiele

### Beispiel B1 (erfindungsgemäß):

Es handelt sich um eine Formulierung mit DSSK, Dehyton PK45, Plantacare 1200 UP und Polyquaternium-10.

Herstellung: Alle Komponenten wurden unter Rühren bei Raumtemperatur in VE-Wasser verlöst. Die Einstellung des pH-Werts erfolgt durch Zugabe von Citronensäure (50 Gew.-%-ige wässrige Lösung). Zusammensetzung gemäß Tabelle 1.

### Beispiel B2 (erfindungsgemäß):

Es handelt sich um eine Formulierung mit DSSK, Dehyton PK45, Plantacare 1200 UP und Polyquaternium-10.

Herstellung: Wie in Beispiel 1 angegeben. Die Zusammensetzung ist Tabelle 1 zu entnehmen.

### Vergleichsbespiel VB1:

Es handelt sich um eine Formulierung mit DSSK, Dehyton PK45, Plantacare 1200 UP. Die Formulierung enthält kein kationisches Polymer.

Herstellung: Wie in Beispiel 1 angegeben. Die Zusammensetzung ist Tabelle 1 zu entnehmen.

**Tabelle 1: Zusammensetzung der Formulierungen und Ergebnisse der Rest-Nasskämmarbeit**

| % Aktivsubstanz | B1 | B2 | VB 1 | VB2 | VB3 | VB4 |
|---|---|---|---|---|---|---|
| DSSK | 4 | 6,7 | 4 | 6,7 | | |
| Dehyton PK 45 | 4 | 2,3 | 4 | 2,3 | 4 | 6 |
| Plantacare 1200 UP | 4 | 3,0 | 4 | 3,0 | 4 | 6 |
| Texapon N70 | | | | | 4 | |
| Ucare Polymer JR-400 | 0,3 | 0,3 | | | 0,3 | 0,3 |
| VE-Wasser | zu 100 | zu 100 | zu 100 | zu 100 | zu 100 | zu 100 |
| | | | | | | |
| pH-Wert | 5,2 | 5,5 | 5,2 | 5,5 | 5,2 | 5,2 |
| | | | | | | |
| Rest-Nasskämmarbeit | 47 % | 43% | 100 % | 107% | 60% | 78% |
| Standardabweichung | +/-3% | +/-5% | +/-7% | +/-8% | +/-6% | +/- 12% |

### Vergleichsbeispiel VB2:

Es handelt sich um eine Formulierung mit DSSK, Dehyton PK45 und Plantacare 1200 UP. Die Formulierung enthält kein kationisches Polymer.

Herstellung: Wie in Beispiel 1 angegeben. Die Zusammensetzung ist Tabelle 1 zu entnehmen.

### Vergleichsbeispiel VB3:

Es handelt sich um eine Formulierung mit Texapon N70, Dehyton PK45, Plantacare 1200 UP und Polyquaternium-10.

Herstellung: Wie in Beispiel 1 angegeben. Die Zusammensetzung ist Tabelle 1 zu entnehmen.

### Vergleichsbeispiel VB4:

Es handelt sich um eine Formulierung mit Dehyton PK45, Plantacare 1200 UP und Polyquaternium-10.

Herstellung: Wie in Beispiel 1 angegeben. Die Zusammensetzung ist Tabelle 1 zu entnehmen.

Die erfindungsgemäßen Formulierungen (B1) und (B2) reduzieren die Nasskämmarbeit deutlich stärker und weisen somit eine deutlich bessere Pflegeleistung am Haar auf, als die entsprechenden Placeboformulierungen (VB1) und (VB2) ohne kationisches Polymer. Sie zeichnen sich außerdem durch eine signifikant bessere Pflegeleistung aus als die konventionelle Ethersulfatbasierte Formulierung (VB3) und die Sulfat-freie Formulierung (VB4), obwohl diese den gleichen Tensidgehalt und gleichen Polymergehalt aufweisen wie (B1) und (B2).

## Patentansprüche

1. Wässrige Tensid-Zusammensetzungen enthaltend
• ein oder mehrere **alpha-Sulfofettsäuredisalze (A)** der allgemeinen Formel (I),
R¹CH(SO₃M¹)COOM² (I),
worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
• ein oder mehrere **Sulfoketone (B),** die ausgewählt sind aus den Verbindungen (F) und den Verbindungen (G),
wobei die Verbindungen (F) die allgemeine Formel (VI)
R⁶CH₂-CO-CHR⁷(SO₃M⁸) (VI),
aufweisen, worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine,
und wobei die Verbindungen (G) die allgemeine Formel (VII)
(SO₃M⁹)R⁸CH-CO-CHR⁹(SO₃M¹⁰) (VII),
aufweisen, worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine,
• ein oder mehrere **kationische Polymere (X)** ausgewählt aus der Gruppe der kationischen Polymere mit den INCI-Bezeichnungen Polyquaternium-6, Polyquaternium-7, Polyquaternium-10, Polyquaternium-11, Polyquaternium-16, Polyquaternium-22, Polyquaternium-24,Polyquaternium-32, Polyquaternium-37, Polyquaternium-39, Polyquaternium-44, Polyquaternium-46, Polyquaternium-47, Polyquaternium-53, Polyquaternium-67, Polyquaternium-68, Polyquaternium-72, Polyquaternium-74, Polyacrylamidopropyltrimonium Chloride, Polymethacrylamidopropyltrimonium Chloride, Acrylamidopropyltrimonium Chloride/Acrylamide Copolymer, Guar Hydroxypropyltrimonium Chloride, Cassia Hydroxypropyltrimonium Chloride und Starch Hydroxypropyltrimonium Chloride,
• ein oder mehrere **Verbindungen (C)** der allgemeinen Formel (III)
R⁴COOM⁵ (III)
wobei der Rest R⁴ einen linearen oder verzweigten Alkyl- oder Alkenylrest mit 7 bis 19 C-Atomen bedeutet und der Reste M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin,
• ein oder mehrere anorganische Salze der Schwefelsäure (D) der allgemeinen Formel (IV)
(M⁶)₂SO₄ (IV)
wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin,
• Wasser,
wobei folgende Maßgabe gilt:
• Sofern die wässrigen Tensid-Zusammensetzungen ein oder mehrere Estersulfonate (E) der allgemeinen Formel (V),
R²CH(SO3M⁷)COOR³ (V)
worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr - und insbesondere zu 90 Gew.-% oder mehr - vorliegen müssen.

2. Zusammensetzungen nach Anspruch 1, wobei der Rest R¹ in der Formel (I) einen gesättigten, linearen Alkylrest mit 10 bis 16 C-Atomen bedeutet, wobei in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 90 Gew.-% oder mehr liegt.

3. Zusammensetzungen nach Anspruch 1 oder 2, wobei die Reste M¹ und M² ausgewählt werden aus der Gruppe H (Wasserstoff) und Na (Natrium).

4. Zusammensetzungen nach einem der Ansprüche 1 bis 3, wobei der pH-Wert der Zusammensetzung auf einen Wert im Bereich von 4 bis 6 liegt.

5. Verwendung der Zusammensetzungen nach einem der Ansprüche 1 bis 4 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.
